# EUROPEAN PATENT APPLICATION

(11) **EP 2 392 323 A1**
(43) Date of publication of application: **07.12.2011**
(21) Application number: 10735887.1
(22) Date of filing: 29.01.2010
(51) Int. Cl.: A61K 31/277, A61K 45/00, A61P 13/08, A61P 35/00, A61P 43/00

(54) **PROSTATE CANCER PROGRESSION INHIBITOR AND PROGRESSION INHIBITION METHOD**

(30) Priority: 30.01.2009 JP 2009019701
(71) Applicant: Kyoto University, Sakyo-ku Kyoto-shi Kyoto 606-8501 (JP); ONO Pharmaceutical Co., Ltd., Osaka-shi, Osaka 541-8526 (JP)
(72) Inventor: OGAWA Osamu, Kyoto-shi Kyoto 606-8501 (JP); TSUJIMOTO Gozoh, Kyoto-shi Kyoto 606-8501 (JP); NAKAMURA Eijiro, Kyoto-shi Kyoto 606-8501 (JP); KAMBA Tomomi, Kyoto-shi Kyoto 606-8501 (JP); SHIMIZU Yosuke, Kyoto-shi Kyoto 606-8501 (JP); TERADA Naoki, Kyoto-shi Kyoto 606-8501 (JP); KANAJI Toshiya, Mishima-gun Osaka 618-8585 (JP); MARUYAMA Takayuki, Mishima-gun Osaka 618-8585 (JP)
(74) Representative: Grünecker, Kinkeldey, Stockmair & Schwanhäusser
(86) International application number: PCT/JP2010/051188
(87) International publication number: WO 2010/087425

(57) **Abstract**

A prostate cancer progression inhibitor comprises 4-(4-cyano-2-{[2-(4-fluoro-1-naphthyl)propanoyl]amino}phenyl)butyric acid, a salt thereof, a solvate thereof, or a prodrug thereof. 4-(4-Cyano-2-{[2-(4-fluoro-1-naphthyl)propanoyl]amino}phenyl)butyric acid is useful as a prostate cancer progression inhibitor because this butyric acid has, for example, a growth inhibiting effect and a hormone responsiveness recovering effect on prostate cancer that has acquired hormone resistance.

## Description

### TECHNICAL FIELD

The present invention relates to (1) a method of inhibiting the progression of prostate cancer, this method being characterized by administering to a mammal an effective dose of 4-(4-cyano-2-{[2-(4-fluoro-1-naphthyl)propanoyl]amino}phenyl)butyric acid, a salt thereof, a solvate thereof, or a prodrug thereof, (2) a prostate cancer progression inhibitor comprising the 4-(4-cyano-2-{ [2-(4-fluoro-1-naphthyl)propanoyl]amino}phenyl)butyric acid, a salt thereof, a solvate thereof, or a prodrug thereof to be used in the foregoing method, and (3) use of 4-(4-cyano-2-{[2-(4-fluoro-1-naphthyl)propanoyl]amino}phenyl)butyric acid, a salt thereof, a solvate thereof, or a prodrug thereof for manufacturing such a progression inhibitor.

### BACKGROUND ART

In the United States, prostate cancer is the leading type of cancer among male patients. In Japan as well, the westernization of the diet in recent years has been accompanied by a rapid rise in the number of patients with prostate cancer.
The options for treating prostate cancer include endocrine therapy, surgery, and radiation therapy. A decision as to which type of treatment to administer is arrived at after taking into account the degree of progression and malignancy of the prostate cancer, the patient's health status and age, and the presence or absence of complications. However, when a patient is initially diagnosed with prostate cancer, the first thing tried is generally endocrine therapy (also called hormone therapy).

In almost all patients, because the prostate cancer at the initial onset of treatment is prostate cancer having sensitivity to male hormones, or what is referred to as hormone-responsive prostate cancer, progression of the prostate cancer can be inhibited by carrying out endocrine therapy which lowers the level of male hormones (also referred to as antiandrogen therapy). Such therapy includes orchiectomy involving removal of the testes, or the administration of drugs such as antiandrogens, female hormone drugs, or luteinizing hormone-releasing hormone (LH-RH) agonists. Generally, when such therapies are carried out on a patient with prostate cancer, shrinkage in the size of the tumor is noted and a marked decline occurs in the PSA (prostate specific antigen) value, which is a tumor marker.

However, in most cases, generally within a half-year to several years of the start of antiandrogen therapy, although the level of male hormones continues to remain low, the prostate cancer begins to grow once again. The acquisition by a prostate cancer of the ability to grow without being affected by the level of male hormones is referred to as "the acquisition of hormone resistance" or "the acquisition of androgen independence." Because no effective treatment exists for the androgen-insensitive prostate cancer, or hormone-resistant (hormone-unresponsive) prostate cancer, which arises as a result, there is an urgent need for the development of new modes of treatment.

On the other hand, 4-(4-cyano-2-{ [2-(4-fluoro-1-naphthyl)propanoyl]amino}phenyl)butyric acid is a compound disclosed in WO 02/16311. Because it has antagonistic effects on EP3 and/or EP4, which are subtypes of the prostaglandin E2 receptor, this compound is known to be effective against cancers (e.g., carcinogenesis, cancer growth, organ metastasis from cancer, bone metastasis from cancer, and hypercalcemia associated with bone metastasis from cancer) (see, for example, Patent Document 1).

In another report implicating a relationship between prostaglandin E2 (PGE2) receptor subtypes and prostate cancer, it has been shown that, by suppressing both EP2 and EP4, it may be possible to lower the level of expression of the EGF receptors and androgen receptors which take part in cancer cell growth (see, for example, Non-Patent Document 1).

It has also been reported that EP2 and EP4 receptor-mediated prostaglandin E effects take part in the process of angiogenesis in prostate cancer (see, for example, Non-Patent Document 2).
Moreover, quinoline derivatives which have antagonistic effects on EP4 are known to be useful in the treatment of prostate cancer (see, for example, Patent Document 2).

However, up until now, there has been no literature which directly relates 4-(4-cyano-2-{[2-(4-fluoro-1-naphthyl)propanoyl]amino}phenyl)butyric acid with prostate cancer, and it has not been known that this compound possesses a prostate cancer progression-inhibiting effect. In particular, no mention or suggestion has been made anywhere of the fact that this compound acts on the process by which prostate cancer acquires hormone resistance, exhibiting such effects as the inhibition of hormone resistance acquisition by hormone-responsive prostate cancer and the recovery of hormone responsiveness of hormone-resistant prostate cancer, or of the fact that this compound acts on the growth process by prostate cancer following the acquisition of hormone resistance by the cancer, exhibiting such effects as the inhibition of hormone-resistant prostate cancer growth alone without influencing the growth of hormone-responsive prostate cancer.

Patent Document 1: WO 02/16311
Patent Document 2: WO 2006/122403

Non-Patent Document 1: Proceedings of the American Association for Cancer Research Annual Meeting, 49, 1111-1112 (2008).
Non-Patent Document 2: Cancer Research, 68(19), 7750-7759 (2008).

Even though endocrine therapy is the first option for treating prostate cancer, in most patients, because a hormone-responsive prostate cancer transforms into a hormone-resistant prostate cancer within at most several years of the start of treatment using endocrine therapy, further endocrine therapy ceases to be effective. In such patients, other treatment modalities do not provide desirable effects. In some cases, radiation treatment alleviates the subjective symptoms to some degree, but is unlikely to cure the patient. In patients with prostate cancer that has acquired hormone resistance, the cancer progresses and grows with the passage of time, then metastasizes, eventually leading to death.

### DISCLOSURE OF THE INVENTION

It is therefore an object of the invention to provide a useful agent which controls the growth of prostate cancer that has acquired hormone resistance and is able to keep the patient from eventually dying due to prostate cancer.

As a result of extensive investigations, the inventors have discovered that, compared with the prostate tissue in hormone-responsive prostate cancer patients, EP4, which is a subtype of prostaglandin E2 receptor, is strongly expressed in the prostate tissue of prostate cancer patients who have acquired hormone resistance; that the forced expression of EP4 in a hormone-responsive prostate cancer cell line results in the acquisition of hormone resistance; and that the compound 4-(4-cyano-2-{[2-(4-fluoro-1-naphthyl)propanoyl]amino}phenyl)butyric acid, which has a antagonistic effect on EP4, inhibits the growth of hormone-resistance prostate cancer even though it does not inhibit the growth of hormone-responsive prostate cancer. Based on these hitherto unknown findings, the inventors conducted further investigations and ultimately discovered that 4-(4-cyano-2-{[2-(4-fluoro-l-naphthyl)propanoyl]amino}phenyl)butyric acid solves the above problems.

Accordingly, this invention relates to:
[1] A hormone resistance acquisition inhibitor for hormone-responsive prostate cancer, which comprises 4-(4-cyan-2-{[2-(4-fluoro-1-naphthyl)propanoyl]amino}phenyl)butyric acid, a salt thereof, a solvate thereof, or a prodrug thereof;
[2] the inhibitor of [1], which is used in combination with an antiandrogen therapy;
[3] the inhibitor of [2], wherein the antiandrogen therapy comprises administration of at least one drug selected from the group consisting of bicalutamide acetate, flutamide acetate, chlormadinone acetate, estramustine phosphate sodium, leuprorelin and goserelin, or orchiectomy;
[4] the inhibitor of [3] which is used in further combination with chemotherapy using cisplatin or docetaxel, with HIFU, or with brachytherapy;
[5] a prostate cancer progression inhibitor comprising 4-(4-cyano-2-{[2-(4-fluoro-1-naphthyl)propanoyl]amino}phenyl)butyric acid, a salt thereof, a solvate thereof, or a prodrug thereof;
[6] the inhibitor of [5], wherein the prostate cancer progression inhibition is the recovery of hormone responsiveness of a hormone-resistant prostate cancer;
[7] the inhibitor of [5], wherein the prostate cancer progression inhibition is the inhibition of growth of a hormone-resistant prostate cancer;
[8] the inhibitor of [5] for use in a hormone-resistant prostate cancer patient;
[9] the inhibitor of [8], wherein the hormone-resistant prostate cancer patient is a prostate cancer patient under antiandrogen therapy which has continued for more than at least six months, and is a patient in which the prostate cancer progression-inhibiting effects by antiandrogen therapy have decreased in comparison with the start of antiandrogen therapy;
[10] a method of inhibiting the acquisition of hormone resistance by hormone-responsive prostate cancer, which comprises administering to a mammal an effective dose of 4-(4-cyano-2-{[2-(4-fluoro-1-naphthyl)propanoyl]amino}phenyl)butyric acid, a salt thereof, a solvate thereof, or a prodrug thereof;
[11] a method of inhibiting the progression of prostate cancer, which comprises administering to a mammal an effective dose of 4-(4-cyano-2-{[2-(4-fluoro-1-naphthyl)propanoyl]amino}phenyl)butyric acid, a salt thereof, a solvate thereof, or a prodrug thereof;
[12] use of 4-(4-cyano-2-{[2-(4-fluoro-1-naphthyl)propanoyl]amino}phenyl)butyric acid, a salt thereof, a solvate thereof, or a prodrug thereof to manufacture a hormone resistance acquisition inhibitor for hormone-responsive prostate cancer;
[13] use of 4-(4-cyano-2-{[2-(4-fluoro-1-naphthyl)propanoyl]amino}phenyl)butyric acid, a salt thereof, a solvate thereof, or a prodrug thereof to manufacture a prostate cancer progression inhibitor;
[14] an agent for recovering hormone responsiveness in a hormone-resistant prostate cancer or for inhibiting progression by a hormone-resistant prostate cancer, which agent comprises 4-(4-cyano-2-{[2-(4-fluoro-1-naphthyl)propanoyl]amino}phenyl)butyric acid, a salt thereof, a solvate thereof, or a prodrug thereof;
[15] the agent of [14] which is used in combination with an antiandrogen therapy;
[16] the agent of [15], wherein the antiandrogen therapy comprises the administration of at least one drug selected from the group consisting of bicalutamide acetate, flutamide acetate, chlormadinone acetate, estramustine phosphate sodium, leuprorelin and goserelin, or orchiectomy;
[17] the agent of [16] which is used in further combination with chemotherapy using cisplatin or docetaxel, with HIFU, or with brachytherapy;
[18] an agent for prolonging the duration of response by antiandrogen therapy, which agent comprises 4-(4-cyano-2-{[2-(4-fluoro-l-naphthyl)propanoyl]amino}phenyl)butyric acid, a salt thereof, a solvate thereof, or a prodrug thereof;
[19] the agent of [18], wherein the antiandrogen therapy comprises the administration of at least one drug selected from the group consisting of bicalutamide acetate, flutamide acetate, chlormadinone acetate, estramustine phosphate sodium, leuprorelin and goserelin, or orchiectomy;
[20] a method of screening for compounds useful in treating hormone-resistant prostate cancer, which method comprises the steps of: (a) forcibly expressing EP4 in a prostate cancer cell line which expresses an androgen receptor and does not express EP4, (b) subcutaneously implanting in nude mice the prostate cancer cell line obtained in step (a), (c) castrating the nude mice obtained in step (b), (d) administering a medium or a test compound to the nude mice obtained in step (c), and (e) comparing tumor diameters or measured values of a tumor marker between a group of the nude mice administered the medium and a group of the nude mice administered the test compound;
[21] an agent for preventing prostate cancer associated with testosterone replacement therapy or an agent for reducing the risk of prostate cancer in testosterone replacement therapy, which agent comprises 4-(4-cyano-2-{[2-(4-fluoro-1-naphthyl)propanoyl]amino}phenyl)butyric acid, a salt thereof, a solvate thereof, or a prodrug thereof;
[22] an agent for preventing prostate cancer or an agent for reducing the risk of prostate cancer, which agent comprises 4-(4-cyano-2-{[2-(4-fluoro-1-naphthyl)propanoyl]amino}phenyl)butyric acid, a salt thereof, a solvate thereof, or a prodrug thereof, and is administered to healthy individuals having a plasma or serum PSA value of at least 0.1 ng/mL;
[23] an agent for prolonging the survival of hormone-resistant prostate cancer patients, which agent comprises 4-(4-cyano-2-{[2-(4-fluoro-1-naphthyl)propanoyl]amino}phenyl)butyric acid, a salt thereof, a solvate thereof, or a prodrug thereof;
[24] a method of lowering, in vitro or in vivo, the androgen-independent rate of proliferation by prostate cancer cells, which method comprises the step of bringing the prostate cancer cells into contact with an EP4 antagonist, wherein the EP4 antagonist is 4-(4-cyano-2-{[2-(4-fluoro-1-naphthyl)propanoyl]amino}phenyl)butyric acid, a salt thereof, a solvate thereof, or a prodrug thereof;
[25] a pharmaceutical composition for lowering the growth rate by hormone-resistant prostate cancer, which composition comprises an EP4 antagonist as the active ingredient, wherein the EP4 antagonist is 4-(4-cyano-2-{[2-(4-fluoro-1-naphthyl)propanoyl]amino}phenyl)butyric acid, a salt thereof, a solvate thereof, or a prodrug thereof;
[26] a method of inhibiting, in vitro or in vivo, the acquisition of hormone resistance by hormone-responsive prostate cancer, which method comprises the step of bringing the hormone-responsive prostate cancer into contact with an EP4 antagonist, wherein the EP4 antagonist is 4-(4-cyano-2-{[2-(4-fluoro-1-naphthyl)propanoyl]amino}phenyl)butyric acid, a salt thereof, a solvate thereof, or a prodrug thereof; and
[27] a pharmaceutical composition for inhibiting the acquisition of hormone resistance by a hormone-responsive prostate cancer, which composition comprises an EP4 antagonist as the active ingredient, wherein the EP4 antagonist is 4-(4-cyano-2-{[2-(4-fluoro-1-naphthyl)propanoyl]amino}phenyl)butyric acid, a salt thereof, a solvate thereof, or a prodrug thereof.

The invention makes it possible to control the progression of hormone-resistant prostate cancer, for which desirable effects have been difficult to achieve by conventional means. Specifically, the invention makes it possible to inhibit the growth of hormone-resistant prostate cancer, to check the acquisition of hormone resistance by hormone-responsive prostate cancer, or to induce the recovery of hormone responsiveness of hormone-resistant prostate cancer. It could not have been foreseen from the conventional art that EP4 takes part in the process of acquiring hormone resistance in prostate cancer, and that 4-(4-cyano-2-{[2-(4-fluoro-1-naphthyl)propanoyl]amino}phenyl)butyric acid, salts thereof, solvates thereof, and prodrugs of any of these, all of which have antagonistic effects on EP4, possess such effects.
Of the effects of the 4-(4-cyano-2-{[2-(4-fluoro-1-naphthyl)propanoyl]amino}phenyl)butyric acid, a salt thereof, a solvate thereof, or a prodrug thereof disclosed in the invention, by pre-administering 4-(4-cyano-2-{[2-(4-fluoro-1-naphthyl)propanoyl]amino}phenyl)butyric acid, a salt thereof, a solvate thereof, or a prodrug thereof, particularly in patients with prostate cancer having hormone responsiveness, the acquisition of hormone resistance by the patient's prostate cancer can be checked or retarded, which is highly useful clinically. By virtue of such an effect, it is possible to use the compound as an adjuvant to antiandrogen therapy, particularly as an agent for prolonging the duration of response by antiandrogen therapy.

### BRIEF DESCRIPTION OF THE DRAWINGS

FIG. 1 is an immunostaining image showing the localization of EP4 in human prostate cancer cells in which EP4 has been forcibly expressed (LNCaP-EP4).
FIG. 2 are diagrams showing the change in tumor volume when human prostate cancer cells in which EP4 has been forcibly expressed (LNCaP-EP4) were implanted in nude mice and the mice were subsequently castrated.
FIG. 3 is a graph showing the effects of 4-(4-cyano-2-{[2-(4-fluoro-1-naphthyl)propanoyl]amino}phenyl)butyric acid (Compound A) on the tumor volume in hormone-resistant prostate cancer xenografts.
FIG. 4 is a graph showing the inhibitory effects of 4-(4-cyano-2-{[2-(4-fluoro-1-naphthyl)propanoyl]amino}phenyl)butyric acid (Compound A) on the acquisition of hormone resistance by hormone-resistant prostate cancer xenografts.

### BEST MODE FOR CARRYING OUT THE INVENTION

In the present invention, 4-(4-cyano-2-{[2-(4-fluoro-1-naphthyl)propanoyl]amino}phenyl)butyric acid (sometimes abbreviated below as "Compound A") refers to the known compound which has formula (A) below 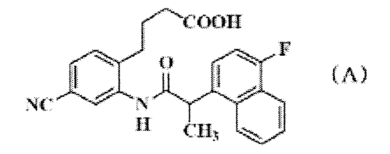 and was disclosed in WO 02/16311.

In this invention, the salt of Compound A is preferably a pharmaceutically acceptable salt. Pharmaceutically acceptable salts are preferably salts which are non-toxic and water-soluble. Suitable salts of Compound A include, for example, alkali metal (e.g., potassium, sodium, lithium) salts, alkaline earth metal (e.g., calcium, magnesium) salts, ammonium salts (e.g., tetramethylammonium salts, tetrabutylammonium salts), and organic amine (e.g., triethylamine, methylamine, dimethylamine, cyclopentylamine, benzylamine, phenethylamine, piperidine, monoethanolamine, diethanolamine, tris(hydroxymethyl)methylamine, lysine, arginine, and N-methyl-D-glucamine) salts.

In the invention, examples of suitable solvates of Compound A include solvates of water and solvates of alcoholic solvents (e.g., ethanol). It is preferable for the solvate to have a low toxicity and be water-soluble. Solvates of Compound A include also solvates of the above-mentioned salts of Compound A.
Compound A may be converted to the above-indicated salts or the above-indicated solvates by a known method.

In the invention, "prodrug of Compound A" refers to a compound which is converted, in vivo, by a reaction involving an enzyme, gastric acid or the like into Compound A. Prodrugs of Compound A are exemplified by compounds in which the carboxyl group on Compound A has been esterified or amidated (e.g., compounds in which the carboxyl group has been ethyl esterified, phenyl esterified, carboxymethyl esterified, dimethylaminomethyl esterified, pivaroyloxymethyl esterified, 1-{(ethoxycarbonyl)oxy{ethyl esterified, phthalidyl esterified, (5-methyl-2-oxo-1,3-dioxolen-4-yl)methyl esterified, 1-{[(cyclohexyloxy)carbonyl]oxy} ethyl esterified or methyl amidated). These compounds may be prepared by a method that is itself known. The prodrug of Compound A may be either a solvate or a non-solvate. Or the prodrug of Compound A may be a compound which changes to Compound A under physiological conditions, such as those mentioned in Iyakuhin no Kaihatsu (The development of medicines), Vol. 7: Bunshi Sekkei (Molecular design) (Hirokawa Shoten, 1990), pp. 163-198). Also, Compound A may be labeled with a radioisotope (e.g., ³H, ¹⁴C, ³⁵S, ¹²⁵I); any atom included on Compound A may be substituted with a corresponding stable isotope (e.g., deuterium (²H), heavy carbon (¹³C), heavy nitrogen (¹⁵N), heavy oxygen (¹⁷O, ¹⁸O).

Compound A, salts thereof, solvates thereof, and prodrugs of any of these can be prepared by a known method, such as the method described in WO 02/16311, a method in general accordance therewith, or the method described in Comprehensive Organic Transformations: A Guide to Functional Group Preparations, 2nd Edition, by Richard C. Larock (John Wiley & Sons, Inc., 1999), or by suitably combining such methods. The reaction product can be purified by an ordinary means of purification, such as distillation under standard pressure or reduced pressure; high-performance liquid chromatography, thin-layer chromatography, or column chromatography using silica gel or magnesium silicate; or washing and recrystallization. Alternatively, if desired, the reaction product may be submitted to treatment such as freeze drying.

In the practice of the invention, Compound A, salts thereof, solvates thereof, or prodrugs of any of these are not limited to a substantially pure single substance, and may include also impurities (such as by-products of the production process, solvents, precursors and the like, or decomposition products) in an amount within a range that is allowable for a bulk drug.
Compound A, a salt thereof, a solvate thereof, or a prodrug thereof prepared by the above method may be used directly as is or, because it is converted in vivo and exhibits an antagonistic effect on EP4, may be used as an EP4 antagonist.

The present invention discloses a method (sometimes referred to below as "the inventive method") for inhibiting the progression of prostate cancer by administering to a mammal (e.g., a human or non-human animal (e.g., monkey, sheep, cow, horse, dog, cat, rabbit, rat, mouse), and preferably to a human (patient), an effective dose of Compound A, a salt thereof, a solvate thereof, or a prodrug thereof; a prostate cancer progression inhibitor (sometimes referred to below as "the inventive agent") which comprises Compound A, a salt thereof, a solvate thereof, or a prodrug thereof, for use in this method; and the use of Compound A, a salt thereof, a solvate thereof, or a prodrug thereof for preparing such a progression inhibitor. In the invention, "progression" signifies the acquisition by prostate cancer of a nature that is undesirable for the mammalian host, such as by growing, metastasizing or acquiring hormone resistance.

In the invention, "prostate cancer" encompasses cancers which arise in the prostate gland, and also all cancers which have metastasized from such a cancer. Generally, most prostate cancers are histologically adenocarcinomas, although squamous cell carcinoma, simple carcinoma and undifferentiated caner are known to be occasionally observed. All of these are encompassed by the term "prostate cancer" as used in the present invention.

Also, the prostate cancer may be at any stage, so long as it is prostate cancer. The stage of the prostate cancer is variously expressed, according to such criteria as the nature and site of the prostate cancer, the symptoms of the patient or marker fluctuations, as, for example, prostate cancer with penetration of prostatic capsule and peripheral infiltration, prostate cancer with osteoblastic bone metastasis, prostate cancer with lymph node metastasis around the external iliac artery, prostate cancer with lymph node metastasis around the aorta, prostate cancer which is asymptotic in the host mammal, prostate cancer which presents symptoms such as dysuria, pollakiuria and hematuria, prostate cancer in which a rise in prostatic acid phosphatase (PAP) is observed, prostate cancer in which a rise in prostate specific antigen (PSA) is observed, and prostate cancer in which a rise in gamma seminoprotein is observed, or combinations thereof. In addition, the stage of prostate cancers is also variously expressed according to known grading methods, including morphology codes for pathological tissue, staging such as T stage, N stage and M stage, or Gleason's grade based on structural atypia of the tumor. The prostate cancer in the present invention may be any of these.

The above prostate cancers are all targets of the present invention; desirable progression inhibiting effects can be obtained in all of these through the invention. Prostate cancer is known to be variously graded as described above, although the prostate cancer progression inhibiting effects obtained by the invention may also be distinguished according to whether the prostate cancer has hormone responsiveness. Specifically, when prostate cancers are broadly divided into "prostate cancer having hormone responsiveness" and "prostate cancer which has acquired hormone resistance," the progression-inhibiting effect of inhibiting the acquisition of hormone resistance can be obtained in "prostate cancer having hormone responsiveness" (hormone-responsive prostate cancer), and the excellent progression-inhibiting effects of recovering hormone responsiveness and suppressing tumor growth can be obtained in "prostate cancer which has acquired hormone resistance" (hormone-resistant prostate cancer).

Here, "the presence or absence of hormone responsiveness" can be judged based on whether changes in the level of male hormones influence the progression of prostate cancer. Specifically, in cases where the progression of prostate cancer is retarded when the level of male hormones decreases, the prostate cancer is classified as being a "prostate cancer having hormone responsiveness" (hormone-responsive prostate cancer). On the other hand, in cases where prostate cancer continues to proceed in spite of a decrease in the level of male hormones, the prostate cancer is classified as being "a prostate cancer which has acquired hormone resistance" (hormone-resistant prostate cancer).

In this invention, "inhibiting the acquisition of hormone resistance by a hormone-responsive prostate cancer" means to keep the hormone-responsive prostate cancer from acquiring the nature of progressing without being affected by changes in the level of male hormones, or to retard the period in which such a nature is acquired. "The recovery of hormone responsiveness of a hormone-resistant prostate cancer" means that the hormone-resistant prostate cancer again acquires the nature of progressing in a manner dependent on quantitative changes in male hormones. Preferred examples of "the recovery of hormone responsiveness of a hormone-resistant prostate cancer" include cases where, as mentioned below, in patients where antiandrogen therapy was no longer effective or the effectiveness had decreased, antiandrogen therapy becomes effective once again or exhibits greater effectiveness.

In the invention, "inhibition in the growth" of prostate cancer refers to a slowing in the rate of propagation by cells of the prostate cancer--that is, a decline in the rate of propagation, and also encompasses preventing such growth altogether. In cases where the prostate cancer is examined in terms of the tumor diameter, a judgment that growth of the prostate cancer is inhibited may be rendered when there is "a retardation in the rate of enlargement in the tumor diameter" or "no observable enlargement in the tumor diameter." In cases where the tumor diameter has shrunk, it may be judged that there is "no observable enlargement in tumor diameter." Alternatively, "inhibition in the growth" of prostate cancer may be observed by using as the indicator the tumor volume computed after measuring the length and breadth of the tumor. For example, in the subsequently described working examples of the invention, the tumor length and breadth were measured with electronic calipers, and the tumor volume was computed using the formula {tumor volume = tumor length × (tumor breadth)² × 0.52}. In cases where the prostate cancer has been observed in terms of the tumor volume, a judgment that growth of the prostate cancer is inhibited may be rendered when there is "a retardation in the rate of enlargement in the tumor volume" or "no observable enlargement in the tumor volume." As when evaluation is carried out based on tumor diameter, in cases where the tumor volume has decreased, it may be judged that there is "no observable enlargement in tumor volume."

In the invention, "antiandrogen therapy" encompasses all means that are capable of lowering the probability that androgens, i.e., male hormones, will come into contact with prostate cancer cells within the body of a prostate cancer patient. That is, such therapy may be a means of lowering the blood concentration of androgen in a prostate cancer patient, or a means of specifically lowering the local concentration of androgen in the prostate gland and peripheral tissue of a prostate cancer patient. As used herein, "androgen" refers generally to male hormones, and includes not only testosterone, but also dihydrotestosterone, dehydroepiandrosterone, androsterone and androstenedione.

In the practice of the invention, examples of antiandrogen therapy include orchiectomy, in which the testes are removed; the administration of drug such as antiandrogen drugs, female hormone drugs or luteinizing hormone-releasing hormone (LH-RH) agonists; and MAB therapy involving the concomitant use of orchiectomy or luteinizing hormone-releasing hormone agonists and antiandrogen drugs. Here, examples of antiandrogen hormone drugs include non-steroidal antiandrogen drugs such as bicalutamide acetate (Casodex®) and flutamide acetate (Odyne®), and steroidal antiandrogen drugs such as chlormadinone acetate (Prostal®). Examples of female hormone drugs include estrogen preparations such as estramustine phosphate sodium (Estracyt®). Examples of luteinizing hormone-releasing hormone agonists include leuprorelin (Leuplin® ) and goserelin (Zoladex®).

In the invention, when the effective dose of Compound A, a salt thereof, a solvate thereof, or a prodrug thereof is used to inhibit the progression of prostate cancer, the route of administration in the mammal serving as the target of administration (e.g., a human or non-human animal, and preferably a human (patient)) may be oral administration or may be parenteral administration. Parenteral administration may be systemic administration such as intra-arterial administration or intravenous administration, or may be local administration such as local injection, percutaneous administration, rectal administration or administration by implantation in tissue around the prostate gland. Intravenous administration may be drip administration, and local injection may involve injection, such as muscular, hypodermic or intradermal injection, at any site.

The dose of Compound A, a salt thereof, a solvate thereof, or a prodrug thereof may be any dose, provided these drugs, when administered in vivo, lack a marked toxicity and exhibit inhibitory effects on the progression of prostate cancer. However, these drugs are generally used in a range of from about 0.01 mg to about 5,000 mg. When the method of administration is changed as noted above, the dose required to obtain the desired effects also changes. Hence, when Compound A, a salt thereof, a solvate thereof, or a prodrug thereof is administered, a dose suitable for the method of administration should be selected.

As a general guide for the dose of Compound A, a salt thereof, a solvate thereof, or a prodrug thereof, when these drugs are administered orally, the dose each time is preferably from about 0.1 mg to about 5,000 mg, more preferably from about 1 mg to about 1,000 mg, and even more preferably from about 3 mg to about 100 mg. When these drugs are administered intra-arterially or intravenously, the dose each time is preferably from about 0.01 mg to about 1,000 mg, more preferably from about 0.1 mg to about 100 mg, and even more preferably from about 1 mg to about 30 mg. When these drugs are administered as local injections, percutaneously, rectally, or by implantation in tissue around the prostate gland, the dose each time is preferably from about 0.01 mg to about 50 mg, and more preferably from about 1 mg to about 10 mg. When a salt of Compound A, a solvate thereof, or a prodrug thereof is used, the amount of Compound A is preferably the above-indicated dose.

In cases where Compound A, a salt thereof, a solvate thereof, or a prodrug thereof is administered by the above methods of administration in a mammal (e.g., a human or non-human animal, and preferably a human (patient)), pharmaceutical compositions which have been rendered into preparations in accordance with the respective mode of administration may be used.

Pharmaceutical compositions used for oral administration include solid preparations for internal use, such as tablets, pills, capsules (hard capsules, soft capsules), powders and granules, and liquid preparations for internal use, such as aqueous solutions, suspensions, emulsions, syrups and elixirs.

Solid preparations for internal use may be prepared in accordance with a conventional method by using Compound A, a salt thereof, a solvate thereof, or a prodrug thereof directly, or by mixing any of the above together with, for example, excipients (e.g., lactose, mannitol, glucose, microcrystalline cellulose, starch), binders (e.g., hydroxypropyl cellulose, polyvinyl pyrrolidone, magnesium aluminometasilicate), disintegrants (e.g., calcium cellulose glycolate), lubricants (e.g., magnesium stearate), stabilizers, and solubilizers (e.g., glutamic acid, aspartic acid). If necessary, a coating agent (e.g., sucrose, gelatin, hydroxypropyl cellulose, hydroxypropylmethyl cellulose phthalate) may be coated thereon, or two or more such layers may be coated thereon. To prepare capsules, filling may be carried out into capsule shells composed primarily of proteins (e.g., gelatin, collagen), polysaccharides (e.g., starch, amylose, polygalacturonic acid, agar, carrageenan, gum arabic, gellan gum, xantham gum, pectin, alginic acid), biodegradable plastics (e.g., polylactic acid, polyhydroxybutyric acid, polyglutamic acid), and hardened fats or oils (e.g., a triglyceride or diglyceride of a medium-chain fatty acid).

Liquid preparations for internal use may be prepared by dissolving, suspending or emulsifying Compound A, a salt thereof, a solvate thereof, or a prodrug thereof in a commonly used diluent (e.g., purified water, ethanol, or a mixture thereof). Moreover, these liquid preparations may include, for example, wetting agents, suspending agents, emulsifying agents, sweeteners, flavoring agents, fragrances, preservatives and buffering agents.

Pharmaceutical compositions used for intra-arterial administration, intravenous administration or local injection may be solutions, suspensions, emulsions, or solid injections that are dissolved or suspended in a solvent at the time of use. These pharmaceutical compositions are prepared by dissolving, suspending or emulsifying Compound A, a salt thereof, a solvate thereof, or a prodrug thereof in a solvent. Solvents that may be used include distilled water for injection, physiological saline, vegetable oils, alcohols such as propylene glycol, polyethylene glycol and ethanol, and combinations thereof. In addition, these pharmaceutical compositions may include also stabilizers, buffers, pH modifiers, dissolving agents, solubilizers, suspending agents, emulsifiers, surfactants, antioxidants, anti-foaming agents, tonicity agents, soothing agents, preservatives, as well as other additives such as those mentioned in Iyakuhin Tenkabutsu Jiten [Dictionary of pharmaceutical additives], edited by International Pharmaceutical Excipients Council Japan, (Yakuji Nippo, 2000). To obtain intravenous preparations for drip instillation, in addition to such additives, use may also be made of ingredients commonly employed in infusions, such as electrolytes (e.g., sodium chloride, potassium chloride, calcium chloride, sodium lactate, sodium dihydrogenphosphate, sodium carbonate, magnesium carbonate), sugars (e.g., glucose, fructose, sorbitol, mannitol, dextran), protein amino acids (e.g., glycine, aspartic acid, lysine), and vitamins (e.g., vitamin B1, vitamin C). These may be sterilized in the final step or may be prepared by way of aseptic operations. Alternatively, a sterile solid preparation such as a freeze-dried product may be manufactured, then sterilized prior to use or dissolved in sterile distilled water for injection or some other solvent.

Pharmaceutical compositions for local injection may be microsphere injections. For information on methods of manufacturing microspheres and methods for using microspheres, if necessary, reference may be made to Maikuro/Nano-kei Kapuseru·Biryüshi no Kaihatsu to Oyo [Development and application of micro/nanocapsules and particles], edited by Masumi KOISHI (CMC Publishing, 2003). Also, for information on release-retarding common physiologically active substances, reference may be made to Doraggu Deribarii·Shisutemu no Jissai [The practice of drug delivery systems], by Kohei MIYAO (Iyaku (Medicine and Drug) Journal, 1986). The above-described pharmaceutical compositions which have been prepared as microsphere injections may be injected intramuscularly, and preferably subcutaneously, so as to carry out the sustained release of Compound A, a salt thereof, a solvate thereof, or a prodrug thereof. Such microsphere injections may be administered intravenously or intra-arterially, as desired.

Pharmaceutical compositions which may be used in percutaneous administration include, for example, liquid sprays, lotions, ointments, creams, gels, sols, aerosols, poultices, plasters and tapes. In these compositions, together with Compound A, a salt thereof, a solvate thereof, or a prodrug thereof, use may be made of, for example, an oil base that is commonly used in external preparations [e.g., vegetable oils (e.g., cottonseed oil, sesame oil, olive oil), waxes (e.g., carnauba wax, beeswax), higher hydrocarbons (e.g., white petrolatum, liquid paraffin, Plastibase), fatty acids (e.g., stearic acid, palmitic acid) and esters thereof, higher alcohols (e.g., cetanol), and silicones (e.g., silicone fluid, silicone rubber)], a water-soluble base [e.g., solutions or high-molecular-weight hydrogels of polyvinyl alcohol, carboxyvinyl polymer or cellulose derivatives, polyethylene glycol (macrogol gels listed in the Pharmacopoeia of Japan), polyethylene glycol-polypropylene glycol copolymers, propylene glycol, 1,3-butylene glycol, ethanol, glycerol], a thickener used in tapes [e.g., synthetic rubber thickeners (e.g., methacrylate copolymers, natural rubber thickeners, synthetic isoprene), silicone polymer thickeners], a film base [e.g., polyethylene, polypropylene, polyethylene-vinyl acetate copolymers, PET, aluminum laminate], a gel base [e.g., dry agar, gelatin, aluminum hydroxide, silicic acid], or an emulsion base obtained by adding a surfactant [e.g., an anionic surfactant (e.g., fatty acids, saponins, fatty acid sarcosides, alcohol sulfuric acid esters, alcohol phosphoric acid esters), cationic surfactant (e.g., quaternary ammonium salts, heterocyclic amines), amphoteric surfactant (e.g., alkyl betaine, lysolecithin), nonionic surfactant (e.g., polyoxyethylene alkyl ethers, polyoxyethylene sorbitan fatty acid esters, sucrose fatty acid esters)] to an oil base and a water-soluble base. Where necessary, commonly used additives may also be added, such as surfactants [e.g., anionic surfactants (e.g., fatty acids, saponins, fatty acid sarcosides, alcohol sulfuric acid esters, alcohol phosphoric acid esters), cationic surfactants (e.g., quaternary ammonium salts, heterocyclic amines), amphoteric surfactants (e.g., alkyl betaine, lysolecithin), nonionic surfactants (e.g., polyoxyethylene alkyl ethers, polyoxyethylene sorbitan fatty acid esters, sucrose fatty acid esters)], thickeners [e.g., cellulose derivatives (e.g., carboxymethyl cellulose), polycarboxylic acids (e.g., polyacrylic acids, methoxy methylene-maleic anhydride copolymers), nonionic water-soluble polymers (e.g., polyvinyl pyrrolidone, polyvinyl alcohol)], stabilizers [e.g., antioxidants (e.g., ascorbic acid, sodium pyrosulfite), chelating agents (e.g., EDTA)], pH adjustors (e.g., phosphate buffers, sodium hydroxide), preservatives [e.g., parabenes, alkyl quaternary ammonium salts (e.g., benzalkonium chloride, benzethonium chloride)], absorption promoters [e.g., fatty acids and esters thereof (e.g., oleic acid, isopropyl myristate), phospholipids (e.g., phosphatidylchlorine), terpenes (e.g., limonene), azacycloalkanes (e.g., Azone™ (Nelson Research))]. These preparations for percutaneous administration which contain Compound A, a salt thereof, a solvate thereof, or a prodrug thereof may be prepared by a conventional method using the various above-mentioned bases, thickeners, and other additives which are added as needed.

Liquid sprays, lotions, sols and aerosols may be produced by dissolving or dispersing Compound A, a salt thereof, a solvate thereof or a prodrug thereof in a solvent such as water, propylene glycol, 1,3-butylene glycol, ethanol or glycerol. Where desired, the above-mentioned additives may also be added.

Ointments and creams may be produced by mixing Compound A, a salt thereof, a solvate thereof or a prodrug thereof with a water-soluble base, an oil base and/or a solvent commonly used in this technical field, such as water or a vegetable oil, optionally adding a surfactant, and subjecting the ingredients to emulsifying treatment. Where desired, the above-mentioned additives may also be added.

Poultices, plasters and tapes may be produced by coating base Compound A, a salt thereof, a solvate thereof or a prodrug thereof and, if desired, a solution containing the above-mentioned thickener (which solution may, if necessary, contain the above-mentioned additives) onto the above-mentioned film base, and optionally subjecting these ingredients to crosslinking treatment or drying treatment.

Gels may be produced by casting Compound A, a salt thereof, a solvate thereof or a prodrug thereof, or a solution containing the salt and the above-described gel base (which solution may also contain, if necessary, the above-described additives) into a mold, and optionally subjecting the cast ingredients to crosslinking treatment or drying treatment.

The pharmaceutical composition for administration by implanting may be Compound A, a salt thereof, a solvate thereof or a prodrug thereof, either used directly as is, or wrapped in a biodegradable sheet and prepared into various shapes according to the intended purpose. For example, such a composition may be rendered into granular, cylindrical, prismatic, sheet-like, disk-like, stick-like, rod-like, spheroidal, particulate or paste-like solid or semisolid preparations.

When manufacturing pharmaceutical compositions for rectal administration, or suppositories, Compound A, a salt thereof, a solvate thereof or a prodrug thereof is rendered into an oil-based or aqueous solid, semisolid or liquid suppository in accordance with a method that is itself known. Examples of oil bases that may be used in such a composition include higher fatty acid glycerides (e.g., cocoa butter, Witepsols (Dynamit Nobel AG)), medium fatty acids (e.g., Miglyols (Dynamit Nobel AG)), and vegetable oils (e.g., sesame oil, soybean oil, cottonseed oil). Examples of aqueous bases that may be used include polyethylene glycols, propylene glycols. Examples of aqueous gel bases that may be used include natural gums, cellulose derivatives, vinyl polymers and acrylic acid polymers.

When these pharmaceutical compositions are used as prostate cancer progression inhibitors, no particular limitation is imposed on the period of administration for the pharmaceutical compositions. The administration of such pharmaceutical composition may be carried out intermittently with suitable drug holidays as desired. In intermittent administration, the drug holidays are preferably at least one day but not more than 30 days. For example, intermittent administration every other day, intermittent administration with two days on and one day off, intermittent administration with five successive days on followed by two days off, or intermittent administration using a common calendar method (for example, in the case of tablets, referred to as "calendar tablets") may be carried out. Alternatively, with regard to administration by implantation in tissue around the prostate gland, for example, because sustained drug effects are expected, administration once per week, once per month, once in three months, once in six months, or once a year is also possible.

The period of administration for the inventive drug is exemplified by, in the case of oral administration or percutaneous administration, from one day to five years, preferably from one day to one year, more preferably from one day to six months, and even more preferably from one day to two months. In the case of intravenous administration, the period of administration may be, for example, from one day to 100 days, preferably from one day to 10 days, and more preferably from one day to one week.

The number of times the drug is administered per day in these periods of administration is exemplified by, when the mode of administration is oral administration or intravenous administration, from one to five times, preferably from one to three times, more preferably one or two times, and most preferably one time. In the case of percutaneous administration, because a blood concentration controlling action can be expected and because medication can be discontinued when the adverse events generally called side effects arise, this may be regarded as an easy-to-use mode of administration for the patient.

In the practice of the invention, Compound A, a salt thereof, a solvate thereof, or a prodrug thereof may be used as a single agent in the form of the above-described pharmaceutical composition, or may be used in combination with other drugs and treatment methods (including surgical treatment) used to treat prostate cancer.

When a pharmaceutical composition comprising Compound A, a salt thereof, a solvate thereof, or a prodrug thereof is used in combination with another drug, administration may be carried out either in the form of a combination drug composed of both ingredients blended in a single preparation, or in the form of concomitant drugs given as separate preparations. Administration as separate preparations includes both simultaneous administration and administration with time lapses therebetween. Examples of other drugs that may be used in combination include various drugs employed in antiandrogen therapy of the sort described above, and anticancer drugs employed in cancer chemotherapy. When the above pharmaceutical composition is used in combination with anticancer drugs, combination with cisplatin or docetaxel is especially preferred.

The drugs mentioned above for use in combination with a pharmaceutical composition comprising Compound A, a salt thereof, a solvate thereof, or a prodrug thereof are illustrative examples only, and are not intended to be limitative. The method of administering such drugs is not subject to any particular limitation, and may be either oral administration or parenteral administration. These drugs may be administered as combinations of any two or more types thereof. Such drugs include not only those which, based on the above-described mechanism, have hitherto been discovered, but also those which will be discovered in the future.

Illustrative examples of surgical therapy carried out in combination with the administration of a pharmaceutical composition comprising Compound A, a salt thereof, a solvate thereof, or a prodrug thereof include surgical therapy such as orchiectomy carried out in antiandrogen therapy as described above, and also surgical therapy carried out to remove prostate cancer tumor tissue, HIFU (High-Intensity Focused Ultrasound prostate treatment system) which destroys prostate cancer tumor tissue in vivo without surgical resection, and brachytherapy in which radiation therapy is carried out with a radiation source that has been implanted in vivo.

Preferred examples of specific combinations include methods where, in a prostate cancer patient in which antiandrogen therapy has been carried out for a period of from a half-year to several years and in which the prostate cancer has acquired hormone resistance, while continuing antiandrogen therapy, administering both a pharmaceutical composition comprising Compound A, a salt thereof, a solvate thereof, or a prodrug thereof, and also cisplatin and/or docetaxel so as to lower the prostate cancer growth rate, then destroying prostate cancer tissue by a surgical therapy such as HIFU.

Aside from being administered in mammals for therapeutic purposes as described above, Compound A, a salt thereof, a solvate thereof, or a prodrug thereof, which is an EP4 antagonist may be experimentally brought into contact with prostate cancer in an in vitro or in vivo test system and used, for example, as a positive control to evaluate whether another EP4 antagonist or a compound having a different pharmacological mechanism has a prostate cancer progression inhibiting effect. In cases where the prodrug of Compound A is used in an in vitro test system, it is necessary to ascertain whether Compound A forms in that test system.

Here, an "in vitro test system," as is understood by persons skilled in the art, is generally any test system which comprises a step in which Compound A, a salt thereof, a solvate thereof, or a prodrug thereof, is brought into contact with prostate cancer ex vivo. For example, it includes the action of adding Compound A, a salt thereof, a solvate thereof, or a prodrug thereof, and having such act upon, for example, prostate cancer tissue that has been removed from the living body, prostate cancer cells that have been isolated from such tissue and cultured, or a prostate cancer cell line.

Also, "in vivo test system," as is understood by persons skilled in the art, is generally any test system which comprises a step in which Compound A, a salt thereof, a solvate thereof, or a prodrug thereof, is brought into contact with prostate cancer in vivo. For example, it includes the action of administering Compound A, a salt thereof, a solvate thereof, or a prodrug thereof, and having such act upon, for example, a prostate cancer model animal or a prostate cancer tumor-bearing model animal.

A screening method for compounds useful in treating hormone-resistant prostate cancer is also disclosed in the present invention. This method comprises the steps of (a) forcibly expressing EP4 in a prostate cancer cell line which expresses an androgen receptor and does not express EP4; (b) subcutaneously implanting in nude mice the prostate cancer cell line obtained in step (a); (c) castrating the nude mice obtained in step (b); (d) administering a medium or a test compound to the nude mice obtained in step (c); and (e) comparing tumor diameters or measured values for a tumor marker between a group of the nude mice administered the medium and a group of the nude mice administered the test compound. Specific examples include the contents disclosed in subsequently described Working Examples 2, 3 and 5. Each of these steps, namely, forced expression of a receptor, subcutaneous implantation in nude mice, castration of the mice, administration of the test compound, and measurement of tumor diameters or tumor markers, may be carried out by known means or with suitable modifications thereto. The compound for administration as a test compound may be any compound having the possibility of being useful in the treatment of hormone-resistant prostate cancer. For example, use may be made of any compound selected from the group of compounds having known EP4 antagonistic effects. That is, use may be made of any compound selected from the group of compounds disclosed in, for example, European Patent Application No. 1,175,889, German Patent Application No. 2,330,307, Japanese Patent Application Laid-open No. 2008-273936, U.S. Patent Application No. 2006/0094742, WO 00/16760, WO 00/21532, WO 01/62708, WO 02/16311, WO 02/20462, WO 02/32422, WO 02/32900, WO 02/50031, WO 02/50032, WO 02/50033, WO 2003/016254, WO 2003/030911, WO 2003/037348, WO 2003/037373, WO 2003/053923, WO 2003/099857, WO 2004/067524, WO 2005/037812, WO 2005/061475, WO 2005/105732, WO 2005/105733, WO 2006/050241, WO 2006/113571, WO 2006/122403, WO 2007/121578, WO 2008/104055 and WO 2008/116304. These compounds may be prepared by the methods described in the specifications of various published patent applications, or by other known methods, such as the methods described in Comprehensive Organic Transformations: A Guide to Functional Group Preparations, 2nd Edition, by Richard C. Larock (John Wiley & Sons, Inc., 1999).

In the "prostate cancer cell line which expresses androgen receptors but does not express EP4" used in the above screening method, the amounts in which the androgen receptors and EP4 are expressed need not be strictly interpreted. For example, it suffices to select a cell line which is a prostate cancer cell line that substantially expresses androgen receptors, and which substantially does not express EP4 or for which the amount of such expression is extremely low, by using a known method of evaluating expression of receptors, such as a fluorescent antibody technique, flow cytometry, Western blot or RT-PCR. One example of such a cell line is LNCaP.

### Pharmacological Tests

Compound A, a salt thereof, a solvate thereof, or a prodrug thereof may be confirmed to have a hormone-resistance acquisition-inhibiting effect on hormone-responsive prostate cancer, or a hormone responsiveness-recovering effect on hormone-resistant prostate cancer, by carrying out, for example, pharmacological tests like those described below. In the following methods, various test conditions are investigated so as to be able to suitably evaluate the pharmacological effects of Compound A, a salt thereof, a solvate thereof, or a prodrug thereof, and improvements which increase the accuracy and/or sensitivity of the evaluation may be added. By selecting a suitable cell line, it is also possible to confirm these effects in vitro.

### Test Example 1: Confirming a Hormone Resistance Acquisition-Inhibiting Effect in Hormone-Responsive Prostate Cancer

As shown in subsequently described Working Example 6, prostate cancer xenografts are created by subcutaneously implanting locally recurrent tissue from prostate cancer patients in nude mice. The mice are castrated, following which they are divided into two groups, only one of which is given Compound A, a salt thereof, a solvate thereof, or a prodrug thereof. The other group is either given nothing or is given only the medium used when administering Compound A, a salt thereof, a solvate thereof, or a prodrug thereof. In the group not given Compound A, a salt thereof, a solvate thereof, or a prodrug thereof, hormone resistance is acquired when the mice are bred for about two months, whereas in the group given Compound A, a salt thereof, a solvate thereof, or a prodrug thereof, either hormone resistance is not acquired or the acquisition of hormone resistance is delayed. Hence, Compound A, a salt thereof, a solvate thereof, or a prodrug thereof can be confirmed to have a hormone resistance acquisition-inhibiting effect on hormone-responsive prostate cancer.

### Test Example 2: Confirming a Hormone Responsiveness-Recovering Effect in Hormone-Resistant Prostate Cancer

As shown in subsequently described Working Example 6, prostate cancer xenografts are created by subcutaneously implanting locally recurrent tissue from prostate cancer patients in nude mice. The mice are raised for about 2 months while continuously being administered effective doses of the above-mentioned drug used in the antiandrogen therapy, and thus made to acquire hormone resistance. After being raised for a period of from several months to one year under loading with Compound A, a salt thereof, a solvate thereof, or a prodrug thereof, the mice are castrated, following which they are raised for another several months and observed. Even though hormone resistance has been acquired, by administering Compound A, a salt thereof, a solvate thereof or a prodrug thereof, a tumor diameter-shrinking effect due to castration is observed. Hence, Compound A, a salt thereof, a solvate thereof, or a prodrug thereof can be confirmed to have a hormone responsiveness-recovering effect on hormone-resistant prostate cancer.
Moreover, Compound A, a salt thereof, a solvate thereof, or a prodrug thereof may also be used as a survival-prolonging drug in hormone-resistant prostate cancer patients. Here, "survival-prolonging" means that, by administering Compound A, a salt thereof, a solvate thereof, or a prodrug thereof, compared with cases in which such is not administered, death as a result of the hormone-resistant prostate cancer is delayed by several weeks, preferably several months, and more preferably several years. This effect can be confirmed by the method shown below. In the following method, as described above, the test conditions may be variously investigated and improvements which increase the accuracy and/or sensitivity of evaluation can be added so as to enable the pharmacological action of Compound A, a salt thereof, a solvate thereof, or a prodrug thereof to be suitably evaluated.

### Test Example 3: Confirming a Survival-Prolonging Action

As shown in subsequently described Working Example 6, prostate cancer xenografts are created by subcutaneously implanting locally recurrent tissue from prostate cancer patients in nude mice. The mice are castrated, following which they were raised for about two months and thus allowed to acquire hormone resistance. The mice are then divided into two groups, only one of which is continuously given Compound A, a salt thereof, a solvate thereof, or a prodrug thereof. The other group is either given nothing or is given only the medium used when administering Compound A, a salt thereof, a solvate thereof, or a prodrug thereof. Earlier deaths are observed in the group not given Compound A, a salt thereof, a solvate thereof, or a prodrug thereof than in the group given such, thereby making it possible to confirm that Compound A, a salt thereof, a solvate thereof, or a prodrug thereof has a survival-prolonging effect.

### Toxicity

The toxicity of Compound A, a salt thereof, a solvate thereof, or a prodrug thereof is very low, and can be judged to be sufficiently safe for use as a pharmaceutical agent.

### Application to Pharmaceuticals

The present invention is characterized by administering an effective dose of Compound A, a salt thereof, a solvate thereof, or a prodrug thereof for the purpose of inhibiting the progression of prostate cancer. The pharmaceutical composition comprising Compound A, a salt thereof, a solvate thereof, or a prodrug thereof which is used in the invention contains Compound A, a salt thereof, a solvate thereof, or a prodrug thereof as the active ingredient, and may be used for the above purpose in a mammal (e.g., a human or non-human animal (monkey, sheep, cow, horse, dog, cat, rabbit, rat, mouse, etc.)). By the systemic administration or local administration, either orally or parenterally, of the pharmaceutical composition in a mammal (e.g., a human or non-human animal, and preferably a human (patient)), particularly via a preferred dosage regimen exemplified in the invention, it is possible to control the progression of hormone-resistant prostate cancer within which desirable effects have been difficult to obtain by conventional means. Specifically, it is possible to inhibit the growth of hormone-resistant prostate cancer, hinder the acquisition of hormone resistance by hormone-responsive prostate cancer, or induce the recovery of hormone responsiveness of hormone-resistant prostate cancer.

Known methods may be used to determine whether prostate cancer progression-inhibiting effects have been obtained by this invention. Prostate cancer diagnosis is generally carried out by some combination of, for example, blood tests, rectal examination, transrectal ultrasonography, biopsies, computed tomography (CT), bone scintigraphy, magnetic resonance imaging (MRI) and medical interviews. Using these means, it is possible to determine whether or not there exist prostate cancer progression-inhibiting effects due to this invention. Moreover, such determinations may be made based on a Gleason score obtained using results from the microscopic examination of tissue collected in biopsies and biochemical tests.

The Gleason score is a major diagnostic criterion for prostate cancer. The method is described in Cancer Chemother. Rep., 50, 125-128 (1966).

One simple method of judging the prostate cancer progression-inhibiting effects is the PSA (prostate specific antigen) test. The PSA test is a blood test; kits which measure the amount of PSA in the blood (preferably in the plasma or serum) are commercially available. For example, this kit may be used to compare the PSA value in the blood (preferably in the plasma or serum) before and after the administration of Compound A, a salt thereof, a solvate thereof, or a prodrug thereof. If the PSA value obtained after administration is similar to or lower than that before administration, a prostate cancer progression-inhibiting effect can be judged to have been obtained by the invention. Of course, because this kit can be used to determine the prostate cancer progression-inhibiting effects not only of Compound A, a salt thereof, a solvate thereof, or a prodrug thereof, but also of antiandrogen therapy, by using this kit to monitor over time the PSA value in the blood (preferably the plasma or serum) of prostate cancer patients, it is possible to identify those patients in which the prostate cancer progression-inhibiting effects due to antiandrogen therapy have decreased relative to the initial stage of antiandrogen therapy.

Compound A, a salt thereof, a solvate thereof, or a prodrug thereof which is disclosed in the present invention may also be used as an agent for preventing prostate cancer or as an agent for reducing the risk of prostate cancer by being administered to prostate cancer high-risk individuals who have a high probability of contracting prostate cancer. Specifically, by pre-administering Compound A, a salt thereof, a solvate thereof, or a prodrug thereof in individuals receiving testosterone replacement therapy or healthy individuals having a plasma or serum PSA value of at least 0.1 ng/ml, the onset of prostate cancer itself is inhibited, enabling use as an agent for preventing prostate cancer or as an agent for reducing the risk of prostate cancer.

Here, testosterone replacement therapy refers to a method for the exogenous administration of testosterone in order to retard or reverse the appearance of symptoms such as reduced libido, decreased muscle mass, increased abdominal fat, reduced bone density, decreased vigor, slowed mathematical and spatial reasoning, and lowered blood cell count which occur due to a decrease in testosterone production within the body.

Patients receiving testosterone replacement therapy and healthy individuals having a plasma or serum PSA value of at least 0.1 ng/mL (preferably at least 0.1 ng/mL, more preferably at least 2 ng/mL, even more preferably at least 4 ng/mL, and most preferably at least 10 ng/mL) are thought to have a high risk of contracting prostate cancer. Even in cases where it has been determined from the results of other tests that such an individual does not have prostate cancer, by pre-administering Compound A, a salt thereof, a solvate thereof, or a prodrug thereof, the very occurrence of prostate cancer can be inhibited, enabling the risk of prostate cancer to be lowered. When administering Compound A, a salt thereof, a solvate thereof, or a prodrug thereof, it is possible to apply, as appropriate, the pharmaceutical compositions and methods of administration thereof disclosed in the invention.

### EXAMPLES

This invention is described in detail below by way of working examples and preparation examples, although the invention is not limited by these examples.
The prostate cancer xenograft model shown in subsequently described Working Example 6 reproduces in an animal model the disease state of a prostate cancer patient. Depending on whether or not the animal is castrated, such a model may be selectively used as a model of a hormone-responsive prostate cancer patient or as a model of a hormone-resistant prostate cancer patient. For example, efficacy in this model when castration has been carried out clearly implies efficacy in hormone-resistant prostate cancer patients.

### Working Example 1: EP4 Immunostaining Using Human Prostatic Tissue

Tissue microarrays were created for prostate cancer tissue collected from 27 prostate cancer patients who had not yet been administered antiandrogen therapy and 31 prostate cancer patients who had acquired hormone resistance due to the administration of antiandrogen therapy, immunostaining using anti-human EP4 polyclonal antibodies (available from MBL) was carried out by a conventional method, and the amount of EP4 expression in the prostate tissue was compared. The prostate cancer tissue used in this test was tissue collected from the prostate glands of full prostatectomy patients, tissue collected in autopsies, or tissue collected during transurethral surgery on locally recurrent tissue.

The immunostaining intensity indicating the amount of EP4 expressed was categorized by physicians specialized in pathology at one of four levels-none, weak, moderate and strong, and using the proportion of the areas on each tissue sample that are more strongly stained as the indicator, was rated at six levels (none, ≤20% weak, >20% weak, ≤20% moderate, >20% moderate, >20% strong). These results are shown collectively in Table 1 below.

**Table 1**

| | Prostate cancer patients not yet given antiandrogen therapy Hormone naive (n = 27) | Prostate cancer patients having acquired hormone resistance from antiandrogen therapy Hormone refractory (n = 31) |
|---|---|---|
| none to ≤20% weak | 10 (37.0%) | 5 (16.1%) |
| >20% weak or ≤20% moderate | 17 (63.0%) | 9 (29.0%) |
| >20% moderate to strong | 0 (0%) | 17 (54.8%) |
| total | 27 | 31 |

| | | |
|---|---|---|
| p = 0.0001 | | |

### Results:

In the prostate cancer tissue of prostate cancer patients who had acquired hormone resistance from the administration of antiandrogen therapy (hormone refractory), the expression of EP4 was elevated compared with that in prostate cancer patients who had not received antiandrogen therapy (hormone naive).

### Working Example 2: Establishment of Human EP4 Forced Expression Prostate Cancer Cells

### (1) Creation of EP4 Expression Vector

A cloning vector (pBluescript-EP4) was cleaved with a restriction enzyme (EcoRI/BamHI), and the excised EP4 gene sequence was integrated into an expression vector (pcDNA3.1(-)), thereby creating an EP4 expression vector (pcDNA3.1-human EP4).

### (2) EP4 Gene Insertion into Human Prostate Cancer Cell Line

The human prostate cancer cell line LNCaP was suspended using a 10% fetal bovine serum (FBS)-containing RPMI-1640 medium, then inoculated at a density of 2.5 × 10⁶ cells per well on a 6 cm dish and cultured for 24 hours. Next, using lipofectamine 2000, gene insertion was carried out by means of the EP4 expression vector (pcDNA3.1-humanEP4) prepared as described above. After 48 hours had elapsed, the resulting cells were cultured using a G418 (1 mg/mL)-containing selection medium, thereby producing monoclonal cells (LNCaP-EP4). Polyclonal cells (LNCaP-mock) were created by carrying out the same procedure using an empty expression vector (pcDNA3.1(-)) instead of the EP4 expression vector.

### (3) Verification of EP4 Expression by Immunostaining

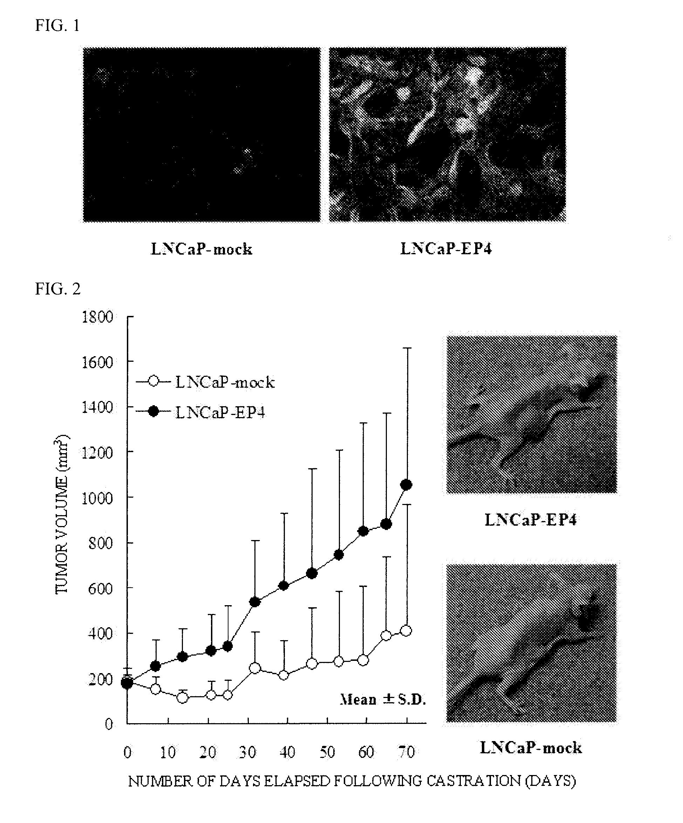

Each clone created as described above was suspended using 10% fetal bovine serum (FBS)-containing RPMI-1640 medium, inoculated at a density of 1.0 × 10⁶ cells per well on a 6 cm dish, and cultured for 48 hours. Fixing with 3.7% paraformaldehyde was then carried out, and cell immunostaining using anti-human EP4 polyclonal antibodies (available from Cayman) was administered by a conventional method. The results are shown in FIG. 1.

### Results:

In LNCaP-EP4 cells, the local presence of EP4 from the cell membrane to the cytoplasm was observed. On the other hand, in LNCaP-mock cells, EP4 expression was not observed.

### Working Example 3: Creation and Evaluation of Tumor-Bearing Mice

The LNCaP-mock cells and LNCaP-EP4 cells prepared in Working Example 2 were respectively mixed (using 1.0 × 10⁷ cells in each case) with 100 µL of Matrigel, and subcutaneously grafted dorsally in nude mice. When the tumor volume reached 100 to 300 mm³, the mice were castrated, following which the tumor volume was measured over time for 70 days. The results are shown in FIG. 2.

### Results:

The tumor volumes were compared 70 days after castration. In mice to which LNCaP-mock cells were grafted, the tumor volume was about twice that at the time of castration, whereas in mice to which LNCaP-EP4 cells were grafted, the tumor volume increased to about 6 times that at the time of castration. Similarly, in mice to which LNCaP-EP4 cells were grafted, a rise in the blood PSA value was also observed. From the above, it was realized that, by inducing the forced expression of EP4 in LNCaP cells, the LNCaP cells acquire a hormone-resistant proliferating ability and a PSA-producing ability.

### Working Example 4: Measurement of EP4 Antagonistic Activity

Mouse EP4-expressing CHO cells prepared in general accordance with the method of Nishigaki et al. (FEBS Lett., 364, 339-341 (1995)) were inoculated to a density of 1 × 10⁵ cells per well on a 24-well microplate and cultured for 2 days. Each well was rinsed with 0.5 mL of minimum essential medium (MEM), following which 0.45 mL of an assay medium (MEM containing 1 mmol/L of IBMX, 1% BSA) was added and incubation at 37°C was carried out for 10 minutes. Next, 0.05 mL of a solution of PGE2 alone, or of this together with Compound A in various concentrations, was reacted at 37°C for 10 minutes, following which 0.5 mL of ice-cooled TCA (10 w/v%) was added, thereby discontinuing the reaction. This reaction mixture, plate and all, was temporarily freeze-dried at -80°C, then thawed, after which the cells were detached with a scraper. Centrifugal separation at 13,000 rpm was carried out for 3 minutes, and the cAMP concentration of the supernatant was measured using a cAMP measurement kit available from Amersham.
The EP4 antagonistic effect of Compound A was computed as the percent inhibition of the reaction at the concentration (100 nM) which exhibits what is substantially the largest cAMP-producing effect with PGE2 alone.

### Results:

The EP4 antagonistic effect of Compound A was 1.3 nM at the IC₅₀ concentration.

### Working Example 5: Study of the Effects of Compound A on LNCaP-EP4 Proliferation and PSA Production

The LNCaP-mock cells and LNCaP-EP4 cells prepared in Working Example 2 were suspended in a 10% fetal bovine serum (FBS)-containing RPMI-1640 culture medium, then inoculated at a density of 1.5 × 10⁵ cells per well of a 6 cm dish and cultured for 24 hours. The medium in each well was then replaced with an androgen-depleted culture solution (10% CSFBS-containing RPBI-1640 medium), following which 10 nM or 100 nM of Compound A was added and culturing was carried out for 6 days, either in the absence or presence of PGE2 (1 µm). The number of these cells was counted, following which the cells were furnished for RNA extraction. The cDNA prepared from the extracted RNA was subjected to a real-time PCR test, and the expression ratio of PSA/GAPDH (glyceraldehyde 3-phosphate dehydrogenase) was determined. These results are shown in Table 2 below (A: data relating to cell growth; B: data relating to PSA expression).

**Table 2**

| (A) Cell Growth | | |
|---|---|---|
| | LNCaP-mock | LNCaP-EP4 |
| PGE2 | 1±0.20 | 1±0.06 |
| PGE2 + Compound A (10 nM) | 1.16±0.09 | 0.88±0.08 |
| PGE2 + Compound A (100 nM) | 1.07±0.20 | 0.57±0.06 |
| (Mean ± S.D.) | | |

| (B) PSA Expression | | |
|---|---|---|
| | LNCaP-mock | LNCaP-EP4 |
| CSFBS | 0.013±0.0018 | 0.156±0.0506 |
| CSFBS + Compound A (10 nM) | 0.009±0.0008 | 0.094±0.0137 |
| CSFBS + Compound A (100 nM) | 0.015±0.0002 | 0.029±0.0023 |
| (Mean ± S.D.) | | |

### Results:

The LNCaP-EP4 cells proliferated with the addition of PGE2 (1 µM). The degree of such growth was a little less than about twice that of the LNCaP-mock. Cell proliferation due to the addition of this PGE2 was concentration-dependently inhibited by the addition of Compound A (see Table 2(A)).

In addition, the change in PSA expression in the absence of PGE2 was evaluated. As a result, when compared with the LNCaP-mock cells, PSA expression was found to be elevated in the LNCaP-EP4 cells. In the same way as above, PSA expression by LNCaP-EP4 cells was concentration-dependently inhibited by Compound A addition (see Table 2(B)).

### Working Example 6: Study of the Effects of Compound A on Prostate Cancer Xenografts

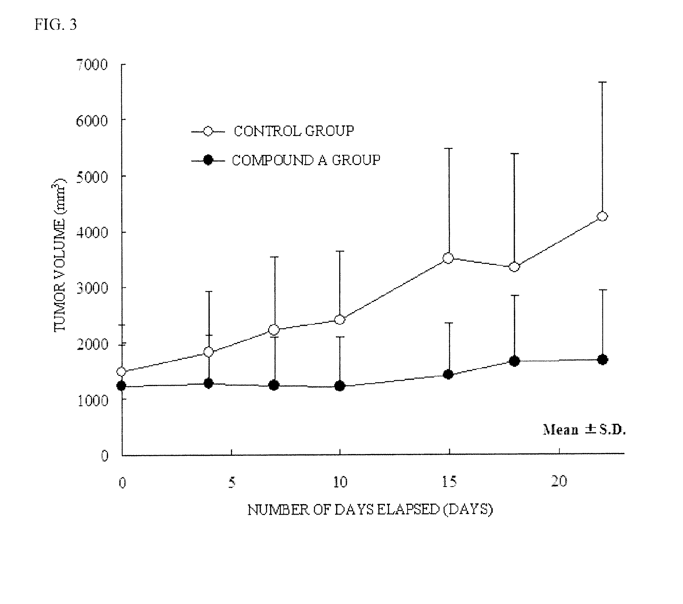

Locally recurrent tissue from prostate cancer patients was grafted subcutaneously in nude mice, thereby creating prostate cancer xenografts (KUCaP/WT). The mice were castrated, then raised for about two months, thereby bringing about the acquisition of hormone resistance. The hormone-resistant prostate cancer xenografts were divided into a control group and a group to be administered Compound A (each group being composed of 5 animals). The mice in each group were intraperitoneally administered distilled water (100 µL/day) or Compound A (20 mg/kg/day) for a period of three weeks, and the tumor volume was observed over time. The results are shown in FIG. 3.

### Results:

In the Compound A group, tumor growth was significantly inhibited compared with the control group. Also, no deaths by the mice in the Compound A group were observed.

To confirm the effects of Compound A under conditions where hormone resistance is not acquired, the same test was carried out without castrating the animals, whereupon no difference in the tumor volumes was observed between the Compound A group and the control group. As a result, the prostate cancer growth inhibiting effects of Compound A were observed only when the prostate cancer was hormone-resistant prostate cancer; Compound A did not show any effects on the growth of hormone-responsive prostate cancer.

### Working Example 7: Study of the Effects of Compound A on the Acquisition of Hormone Resistance by Hormone-Responsive Prostate Cancer

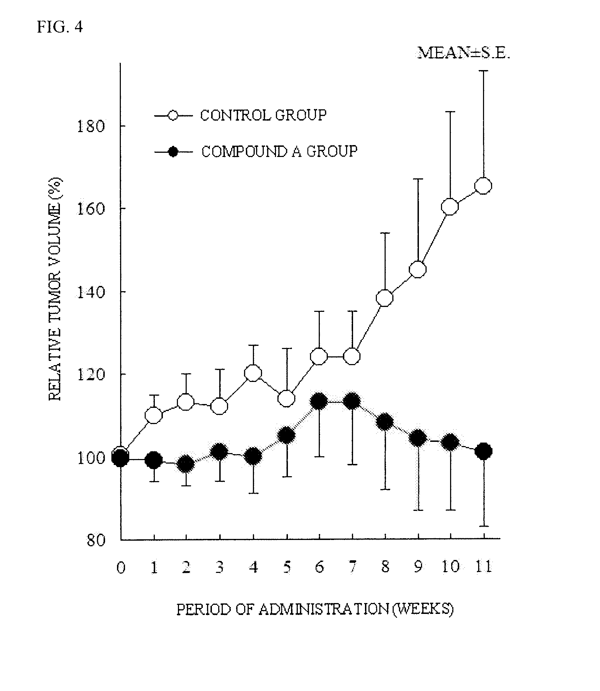

Locally recurrent tissue from prostate cancer patients was grafted subcutaneously in nude mice, thereby creating prostate cancer xenografts (KUCaP/WT). The mice were raised for about two months, then castrated, then raised for about one more month, after which they were divided into two groups-a control group and a Compound A group. Of these, those mice in which the tumor volume did not exceed 2,500 mm³ (5 animals per group) were selected for dosing. For a period of 11 weeks, the animals in the control group were orally administered distilled water (10 mL/kg/day), the animals in the Compound A group were orally administered Compound A (100 mg/10 mL/kg/day during weeks 1 to 4, and 50 mg/10 mL/kg/day during week 5 and thereafter), and the tumor volumes were observed each week. The changes in the tumor volumes are shown in FIG. 4. In the diagram, the tumor volumes on each day of measurement are indicated as a volumetric ratio based on a value of 100% for the tumor volume on the day that administration was started (the day the animals were divided into groups).

### Results:

In the control group, an increase in tumor volume was observed as of week 6 of administration, whereas in the Compound A group, even at week 11 of administration, the tumor volume was similar to that at the time administration was started. The regrowth of cancer cells observed following castration is thought to be due to the acquisition of hormone resistance. Because cancer cell regrowth was inhibited by the administration of Compound A, Compound A was confirmed to have a hormone resistance acquisition-inhibiting effect on hormone-responsive prostate cancer.

### Formulation Example 1

Each of the following ingredients was mixed together by a conventional method and tableted to give 100,000 tablets containing 50 mg of active ingredient per tablet: 4-(4-cyano-2-{[2-(4-fluoro-1-naphthyl)propanoyl]amino}phenyl)butyric acid (5.0 kg), carboxylmethyl cellulose calcium (disintegrant) (0.2 kg), magnesium stearate (lubricant) (0.1 kg) and microcrystalline cellulose (4.7 kg).

### Formulation Example 2

Each of the following ingredients was mixed together by a conventional method, then passed through a dust-removing filter, filled into ampules in amounts of 5 mL per ampule, and heat sterilized in an autoclave to give 100,000 ampules containing 20 mg of active ingredient per ampule: 4-(4-cyano-2-{[2-(4-fluoro-1-naphthyl)propanoyl]amino}phenyl)butyric acid (2.0 kg), mannitol (20 kg), distilled water (500 L).

### INDUSTRIAL APPLICABILITY

The prostate cancer progression inhibitor comprising 4-(4-cyano-2-{[2-(4-fluoro-1-naphthyl)propanoyl]amino}phenyl)butyric acid, a salt thereof, a solvate thereof, or a prodrug thereof which is disclosed in the invention is safe and has growth-inhibiting, hormone responsiveness-recovering and other effects on hormone-resistant prostate cancer that has been intractable to the existing medical art, and is thus highly useful as a pharmaceutical agent. By administering the inventive agent from the stage of prostate cancer having hormone responsiveness, the acquisition of hormone resistance can be checked or retarded, thus enabling use also as an adjuvant to antiandrogen therapy, or as an agent for prolonging the duration of response in antiandrogen therapy.

## Claims

1. A hormone resistance acquisition inhibitor for hormone-responsive prostate cancer, which comprises 4-(4-cyano-2-{[2-(4-fluoro-1-naphthyl)propanoyl]amino}phenyl)butyric acid, a salt thereof, a solvate thereof, or a prodrug thereof.

2. The inhibitor according to claim 1, which is used in combination with an antiandrogen therapy.

3. The inhibitor according to claim 2, wherein the antiandrogen therapy comprises administration of at least one drug selected from the group consisting of bicalutamide acetate, flutamide acetate, chlormadinone acetate, estramustine phosphate sodium, leuprorelin and goserelin, or orchiectomy.

4. The inhibitor according to claim 3, which is used in further combination with chemotherapy using cisplatin or docetaxel, with HIFU, or with brachytherapy.

5. A prostate cancer progression inhibitor comprising 4-(4-cyano-2-{[2-(4-fluoro-1-naphthyl)propanoyl]amino}phenyl)butyric acid, a salt thereof, a solvate thereof, or a prodrug thereof.

6. The inhibitor according to claim 5, wherein the prostate cancer progression inhibition is the recovery of hormone responsiveness of a hormone-resistant prostate cancer.

7. The inhibitor according to claim 5, wherein the prostate cancer progression inhibition is inhibition of growth of a hormone-resistant prostate cancer.

8. The inhibitor according to claim 5, for use in a hormone-resistant prostate cancer patient.

9. The inhibitor according to claim 8, wherein the hormone-resistant prostate cancer patient is a prostate cancer patient under antiandrogen therapy which has continued for more than at least six months, and is a patient in which prostate cancer progression-inhibiting effects by antiandrogen therapy have decreased in comparison with the start of antiandrogen therapy.

10. A method of inhibiting acquisition of hormone resistance of hormone-responsive prostate cancer, which comprises administering to a mammal an effective dose of 4-(4-cyano-2-{[2-(4-fluoro-l-naphthyl)propanoyl]amino}phenyl)butyric acid, a salt thereof, a solvate thereof, or a prodrug thereof.

11. A method of inhibiting progression of prostate cancer, which comprises administering to a mammal an effective dose of 4-(4-cyano-2-{[2-(4-fluoro-1-naphthyl)propanoyl]amino}phenyl)butyric acid, a salt thereof, a solvate thereof, or a prodrug thereof.

12. Use of 4-(4-cyano-2-{[2-(4-fluoro-1-naphthyl)propanoyl]amino}phenyl)butyric acid, a salt thereof, a solvate thereof, or a prodrug thereof to manufacture a hormone resistance acquisition inhibitor for hormone-responsive prostate cancer.

13. Use of 4-(4-cyano-2-{[2-(4-fluoro-1-naphthyl)propanoyl]amino}phenyl)butyric acid, a salt thereof, a solvate thereof, or a prodrug thereof to manufacture a prostate cancer progression inhibitor.
